# EUROPEAN PATENT SPECIFICATION

(11) **EP 1 004 297 B1**
(45) Date of publication and mention of the grant of the patent: **11.01.2006**
(21) Application number: 99122470.0
(22) Date of filing: 11.11.1999
(51) Int. Cl.: A61K 9/20, A61K 9/32, A61K 31/606

(54) **Pharmaceutical compositions for oral administration containing a gastroresistant coating based on acrylic polymers**
Pharmazeutische Zusammensetzungen zur oralen Verabreichung, die einen magensaftresistenten Überzug auf der Basis von Acrylatpolymeren enthalten
Composition pharmaceutique pour administration orale, contenant un enrobage gastrorésistant à base de polymères acryliques

(30) Priority: 13.11.1998 IT MI982467
(43) Date of publication of application: 31.05.2000
(73) Proprietor: LABORATORIO FARMACEUTICO C.T. S.r.l., 18038 Sanremo (Imperia) (IT)
(72) Inventor: Cacciaglia, Roberto, 18014 Ospedaletti (Prov. of Imperia) (IT); Cantarelli, Anna Maria, 29010 Roveleto di Cadeo (Prov. of Piac.) (IT); Tavazzi, Sandra, 40134 Bologna (IT); Ugolotti, Loredana, 18011 Arma di Taggia (Prov. of Imperia) (IT)
(74) Representative: Gervasi, Gemma

(56) References cited:
- WO-A-83/00435
- US-A- 5 644 011
- THE ROYAL PHARMACEUTICAL SOCIETY: "Martindale 31th edition" 1996 , JAMES EF REYNOLDS XP002131821 * page 1371 *

## Description

### Field of the invention

The present invention relates to pharmaceutical compositions, based on mesalazine or pharmaceutically acceptable salts thereof, to be administered by the oral way in tablet form, comprising a gastroresistant coating and being useful for the treatment of inflammatory or irritative intestinal diseases.

### State of the art

There are a number of active ingredients efficacious in intestinal diseases, which however, cannot be administered *per os*, as they are inactivated before reaching the intestine. Furthermore, should said substances cause gastric intolerance and ulceration, their transit through the stomach produces untoward side effects.

For example, mesalazine, i.e. 5-aminosalicylic acid, is a well known drug effective in the treatment of inflammatory intestinal diseases, in particular ulcerative colitis and Crohn's disease, which, however, cannot be administered by the oral way, being absorbed and inactivated before reaching the terminal portion of the intestine, where inflamed and irritated zones are concentrated.

This is one reason why a number of compositions suitable for rectal administration have been developed, whose active ingredients directly act on the mucous membrane of the colon, without being degraded in the stomach. However, patients find the use of said formulations somehow uncomfortable and less acceptable than oral formulations. Moreover, the colon right side cannot be reached and efficaciously treated by rectal formulations.

Alternatively, investigations have been conducted on formulations suitable for oral administration, providing a delayed release of the active ingredient directly in the intestine.

Exemplary preparations of this type are described in European Patent Application No. EP 366,621 (Istituto De Angeli SpA). The pharmaceutical composition of this patent application, designed for oral administration, provides a delayed release of active ingredients suitable for the treatment of intestinal diseases in the colon, by means of a three-layer polymeric coating.

The PCT application No. WO 8,300,435 (Tillott JB Ltd.) discloses pharmaceutical compositions in capsule or tablet form for oral administration, useful for the treatment of intestinal diseases. Said compositions comprise a polymeric coating, thick enough to prevent their degradation before reaching the colon.

Therefore, the preparations known in the art provide an adequate drug release only when they are covered with multi-layer or very thick single-layer coatings; consequently drug release is not only "delayed", but also "controlled", over a very long period of time.

The US Patent No. 5,644,011 discloses a copolymerisate useful as coating agent for pharmaceutical forms, which dissolves in the deeper intestine and is made up of:
A) 10 to 25 wt. % methacrylic acid;
B) 40 to 70 wt. % methyl acrylate; and
C) 20 to 40 wt. % methyl methacrylate; based on the total weight of the copolymerisate.

Therefore, the need for delayed-release oral pharmaceutical compositions overcoming the disadvantages of the aforesaid preparations is deeply felt.

### Summary

The Applicant has now surprisingly found pharmaceutical compositions based on mesalazine or pharmaceutically acceptable salts thereof, suitable for oral administration in tablet form, containing a single-layer filming coating soluble at pH >7.0, which remains intact until it reaches the colon and prevents the drug release in the stomach and small intestine.

It is an object of the present invention to provide pharmaceutical compositions for oral administration in tablet form, containing mesalazine or pharmaceutically acceptable salts thereof as the active ingredient, comprising:
a) a nucleus comprising the active ingredient and maltodextrin in a quantity between 10% and 20% by wt. of the total weight of the active ingredient, and
b) a filming coating comprising a methacrylic acid/methyl acrylate/methyl methacrylate terpolymer.

The pharmaceutical compositions of the present invention are especially suitable for the treatment of inflammatory or irritative intestinal diseases.

Characteristics and advantages of the pharmaceutical compositions of the invention and of the relevant preparation process are illustrated in detail in the description reported below.

### Brief description of the drawing

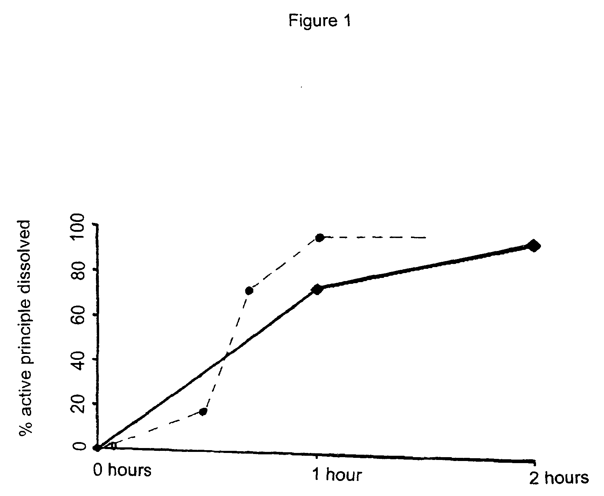

Fig. 1 shows the tablet release profile at pH 7.5, obtained by plotting the % amount of dissolved active ingredient vs time.

### Detailed description of the invention

Nucleus a) contains maltodextrin, which, compared with substances of the prior art exhibiting analogous dispersing properties, e.g. lactose and microcrystalline cellulose, is better tolerated by patients suffering from the aforementioned intestinal pathologies. In fact, said patients are often intolerant to lactose and must live on diets poor in fibre and cellulose.

Typically, according to the present invention, the quantity of maltodextrin in nucleus a) is between 10% and 20% by wt., and more preferably 16% by wt., of the total weight of the active ingredient.

The composition of the nucleus according to the present invention may optionally contain further excipients selected out of binders, wetting agents, anticaking agents, lubricants and mixtures thereof.

Preferably, the binder is polyvinylpyrrolidone, the wetting agent is sodium lauryl sulphate, the anticaking agent is sodium starch glycolate, the lubricants are magnesium stearate, vegetable magnesium stearate, talc, and mixtures thereof.

In a preferred embodiment of the present composition, the total quantity of excipients is between 30% and 40% by wt. of the weight of the active ingredient.

According to the present invention, nucleus a) is coated with filming coating b) comprising an acrylic terpolymer, having the following repeating units in the chain:

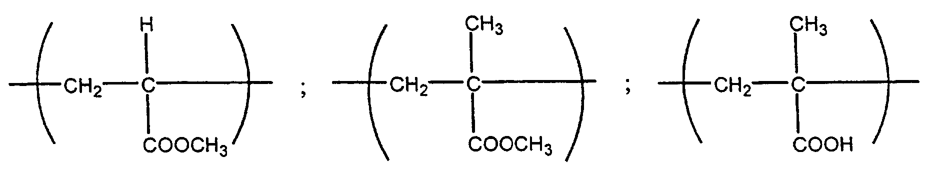

Typically, the quantity of methyl acrylate monomeric unit is between 60% and 70% by wt., the quantity of methyl methacrylate is between 20% and 30% by wt., and the quantity of methacrylic acid is between 5% and 15% by wt. in respect of the total terpolymer weight.

The methyl acrylate/methyl methacrylate/methacrylic acid ratio is preferably equal to 65:25:10 by weight.

The filming coating of the invention is preferably prepared from a product available under the trademark Eudragit 4110 D ® from Rofarma Italia s.r.l., consisting of a 30% aqueous dispersion of the aforesaid methacrylic acid/methyl acrylate/methyl methacrylate terpolymer.

The quantity of acrylic terpolymer in the coating is preferably between 45% and 55% by wt., and more preferably between 49% and 50% by wt., of the total weight of the coating.

The filming coating of the invention may contain plasticising agents, and optionally lubricants, colouring agents, polishing agents and opacifying agents, too.

In a preferred embodiment, the plasticising agent is triethyl citrate, preferably in a 1:20 weight ratio to the acrylic terpolymer.

Typically, talc is used as lubricant for the present coating, red and yellow iron oxide are used as colouring agents, and titanium dioxide is used to increase the hiding power.

Polyethylene glycol 6000 is optionally used as a polishing agent of painted tablets. The quantity of filming coating is preferably between 5.0% and 7.5% by wt. of the total weight of the nucleus. Its thickness is preferably lower than 60 µm, more preferably ranging from 25 to 50 µm, and most preferably equal to 30 µm.

The filming coating of the invention is completely insoluble at pH ≤ 6.0 and dissolves completely at pH >7.0.

It is a further object of the present invention to provide a process for the preparation of the aforesaid pharmaceutical compositions.

Said process comprises the following steps:
i) mixing the active ingredient with maltodextrin, wet granulating the resulting mixture with a solution containing binders and wetting agents, and drying the granular mass obtained;
ii) compressing the granular mass as obtained in step i), optionally added with excipients selected out of binders, wetting agents, anticaking agents, lubricants and mixtures thereof;
iii) adding a 30% aqueous dispersion of methacrylic acid/methyl acrylate/methyl methacrylate terpolymer with an NaOH aqueous solution until neutrality, then with the plasticising and polishing agents; optionally adding said dispersion with an aqueous suspension of colouring agents, optionally containing a lubricant and an opacifying agent, to give the aforementioned filming mixture;
iv) spray coating the tablets as obtained in step ii) above with the mixture prepared as described in step iii).

The compositions in tablet form being the object of the present invention contain the active ingredient in a quantity ranging from 200 to 800 mg. According to preferred embodiments, said tablets may contain 250, 400, 500, and 800 mg of the active ingredient.

The following example is conveyed by way of indication of the present invention.

### Example

### Composition of each tablet

| | |
|---|---|
| *Active ingredient:* | |
| 5-aminosalicylic acid | 400 mg |

| | |
|---|---|
| *Excipients:* | |
| maltodextrin | 64 mg |
| polyvinylpyrrolidone | 20 mg |
| sodium lauryl sulphate | 4 mg |
| sodium starch glycolate | 32 mg |
| talc | 10 mg |
| magnesium stearate | 8 mg |

| | |
|---|---|
| *Coating:* | |
| methyl acrylate/methyl methacrylate/methacrylic acid terpolymer | 18 mg |
| sodium hydroxide | 0.24 mg |
| triethyl citrate | 0.9 mg |
| iron oxide red | 3 mg |
| iron oxide yellow | 0.5 mg |
| titanium dioxide | 3 mg |
| talc | 9 mg |
| polyethylene glycol 6000 | 1.8 mg |

Mesalazine (22 kg) and maltodextrin (3.52 kg) were sieved through a vibrating screen and directly fed to a granulator, where they were added with a binding suspension (5.82 kg) previously prepared by mixing sodium lauryl sulphate (0.66 kg) and polyvinylpyrrolidone K 30 (3.3 kg) in pufiried water (13.5 kg), over a period of 1 hr.

The mixture of mesalazine with maltodextrin was blended with the binding suspension at 25° to 27°C for 2 min. Purified water (1 kg) was added and blending was continued.

The product obtained was forced through Quadro Comil's net, mod. Q 050/50. The resulting granular mass was dried at 50°C for 1.5 hr to a residual moisture content of 1.2% to 2.0%.

The granular mass was mixed with sodium starch glycolate (1.76 kg), talc (0.55 kg), and magnesium stearate (0.44 kg). The mixture obtained was compressed and formed into 55,000 tablets, each containing 400 mg mesalazine.

The filming coating was prepared as follows: Eudragit 4110 D ® (9.9 kg), as a 30% aqueous dispersion, was brought to neutrality by addition of a 1 N NaOH solution (0.99 l) under gentle stirring, which stirring was continued for an additional ½ hr; then triethyl citrate (0.149 kg) and polyethylene glycol 6000 (Carbowax 6000) (0.99 kg) were added.

The resulting dispersion was slowly added with talc (1.485 kg), titanium dioxide (0.495 kg), iron oxide red (0.495 kg), and iron oxide yellow (0.085 kg), suspended in purified water (15 kg).

Considering that process losses equalled 10% approx., the quantity of paint obtained as described above was sufficient to coat 150,000 tablets.

Tablets prepared in advance were filmed by spraying said paint in a quantity adequate for covering each tablet with a 30 µm thick coating, equalling 6.73% by wt. of the weight of the nucleus.

### Release tests

Although the compositions of the invention are coated with a single film layer, their release profile is unexpectedly better than that of mesalazine-based compositions of the prior art.

According to an *in vitro* test, the filming coating of the present invention does not dissolve even after 2 hrs at pH ≤ 6.0; at pH 7.0, it dissolves completely in an hour's time. At pH values of 6.5 to 7.0, the coating of the invention does not disintegrate in the first hour and disintegrates by 30% max. in the second hour.

The two *in vitro* release tests illustrated hereinbelow were conducted, according to a U.S. Pharmacopoeia's (USP) dissolution test, on film-coated tablets of the invention prepared as described in the Example. In said tests, the active ingredient dosage was determined on a spectrophotometer (Beckman DU64) at 300 nm, provided with an automatic sampling system and a quartz cell with side of 0.2 cm.

### Test No. 1

The active ingredient dosage in the dissolution medium was determined under the following operating conditions:
dissolution medium: simulated gastric fluid, pH 1.2
speed of rotation: 100 rpm
temperature: 37°C

At said pH value, no tablet disgregation took place even after 2 hrs.

This test provided evidence of the gastric resistance brought about by the coating of the invention, completely insoluble at acid pH.

### Test No. 2

The active ingredient dosage was determined 30, 45, and 60 min after placing the tablet in a dissolution medium under the following conditions:
dissolution medium: buffer, pH 7.5
speed of rotation: 100 rpm
temperature: 37°C.

Test No. 2 was also conducted, under the same operating conditions, on mesalazine-based gastroresistant tablets of the prior art, in particular on tablets available under the trademark Asacol ® (800 mg) from Giuliani S.p.A.

The quantity of dissolved active ingredient measured at different times and expressed as % by wt. of the total weight of the tablet, is as follows:

| **Time (min)** | **Active ingredient dissolved (%)** | |
|---|---|---|
| | **Asacol ®** | **Tablets of the invention** |
| 30 | --- | 17.0 |
| 45 | --- | 73.4 |
| 60 | 74.6 | 97.3 |
| 120 | 96.9 | --- |

The release profile related to the coating of Asacol ® tablets (full line) and to the coating of tablets of the invention (broken line), respectively, is shown in Fig. 1. As may be seen, by comparing the two release profiles, the filming coating according to the present invention improves the active ingredient release at the pH value of the intestinal terminal portion: the tablets of the invention liberate almost the whole amount of drug after a one-hour residence at said pH, whereas the tablets of the prior art need two hours to achieve the same result.

## Claims

1. Pharmaceutical compositions for oral administration in tablet form, containing mesalazine or pharmaceutically acceptable salts thereof, as the active ingredient, comprising:
a) a nucleus comprising the active ingredient and maltodextrin in a quantity between 10% and 20% by wt. of the total weight of the active ingredient, and
b) a filming coating containing as the sole filming polymer a terpolymer, having the following methyl acrylate, methyl methacrylate, methacrylic acid monomeric units in the chain:

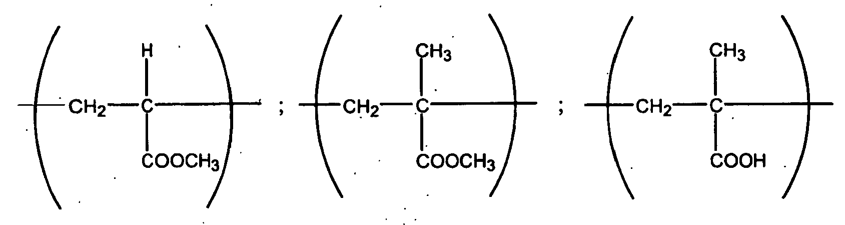

2. The pharmaceutical compositions as claimed in claim 1 for the treatment of inflammatory or irritative intestinal diseases.

3. The pharmaceutical compositions as claimed in claim 1, wherein said nucleus a) comprises maltodextrin in a quantity of 16% by wt. of the total weight of the active ingredient.

4. The pharmaceutical compositions as claimed in claim 1, wherein said nucleus a) comprises excipients selected from the group consisting of binders, wetting agents, anticaking agents, lubricants and mixtures thereof.

5. The pharmaceutical compositions as claimed in claim 4, wherein the binder is polyvinylpyrrolidone, the wetting agent is sodium lauryl sulphate, the anticaking agent is sodium starch glycolate, the lubricants are magnesium stearate, vegetable magnesium stearate, talc, and mixtures thereof.

6. The pharmaceutical compositions as claimed in claim 1, wherein the total quantity of excipients in nucleus a) is between 30% and 40% by wt. of the weight of the active ingredient.

7. The pharmaceutical compositions as claimed in claim 1, wherein the quantity of said filming coating b) is between 5.0% and 7.5% by wt. of the total weight of the nucleus.

8. The pharmaceutical compositions as claimed in claim 1, wherein said filming coating b) has a thickness lower than of 60 µm.

9. The pharmaceutical compositions as claimed in claim 8, wherein said filming coating b) has a thickness ranging from 25 to 50 µm.

10. The pharmaceutical compositions as claimed in claim 8, wherein said filming coating b) has a thickness of 30 µm.

11. The pharmaceutical compositions as claimed in claim 1, wherein, in the terpolymer of filming coating b), the quantity of methyl acrylate is between 60% and 70% by wt., the quantity of methyl methacrylate is between 20% and 30% by wt., and the quantity of methacrylic acid is between 5% and 15% by wt. in respect of the total terpolymer weight.

12. The pharmaceutical compositions as claimed in claim 11, wherein the methyl acrylate/methyl methacrylate/methacrylic acid ratio by weight in said terpolymer is 65:25:10.

13. The pharmaceutical compositions as claimed in claim 1, wherein said filming coating b) contains excipients selected from the group consisting of plasticising, polishing, colouring, and opacifying agents and mixtures thereof.

14. The pharmaceutical compositions as claimed in claim 13, wherein said filming coating b) contains triethyl citrate as plasticising agent, polyethylene glycol 6000 as polishing agent, titanium dioxide as opacifying agent, talc as lubricant, while the colouring agents are selected from the group consisting of iron oxide red, iron oxide yellow, and mixtures thereof.

15. The pharmaceutical compositions as claimed in claim 14, wherein said filming coating b) contains as the plasticising agent triethyl citrate in a 1:20 ratio by wt. to the methacrylic acid/methyl acrylate/methyl methacrylate terpolymer.

16. Process for the preparation of pharmaceutical compositions for oral administration in tablet form, containing mesalazine or pharmaceutically acceptable salts thereof as the active ingredient, comprising:
a) a nucleus comprising the active ingredient and maltodextrin in a quantity between 10% and 20% by wt. of the total weight of the active ingredient, and
b) a filming coating containing as the sole filming polymer a methyl acrylate/methyl methacrylate/methacrylic acid terpolymer,
said process comprising the following steps:
i) mixing the active ingredient with maltodextrin, wet granulating the resulting mixture with a solution containing binders and wetting agents, and drying the granular mass obtained;
ii) compressing the granular mass as obtained in step i), optionally added with excipients selected from the group consisting of binders, wetting agents, anticaking agents, lubricants and mixtures thereof;
iii) adding a 30% aqueous dispersion of methacrylic acid/methyl acrylate/methyl methacrylate terpolymer with the plasticising and polishing agent; optionally adding said dispersion with an aqueous suspension of colouring agents, optionally containing a lubricant and an opacifying agent, to give the aforementioned filming mixture;
iv) spray coating the tablets as obtained in step ii) above with the mixture prepared as described in step iii).

17. The process as claimed in claim 16, wherein the 30% aqueous dispersion of terpolymer as per step iii) is previously brought to neutrality by adding an NaOH aqueous solution.

## Revendications

1. Compositions pharmaceutiques pour l'administration orale sous forme de comprimé, contenant de la mésalazine ou des sels de celle-ci acceptables du point de vue pharmaceutique, en tant que principe actif, comprenant :
a) un noyau comprenant le principe actif et de la maltodextrine en une quantité comprise entre 10% et 20% en poids du poids total du principe actif, et
b) un enrobage filmogène contenant en tant qu'unique polymère filmogène un terpolymère ayant dans la chaîne les unités monomères acrylate de méthyle, méthacrylate de méthyle, acide méthacrylique suivantes :

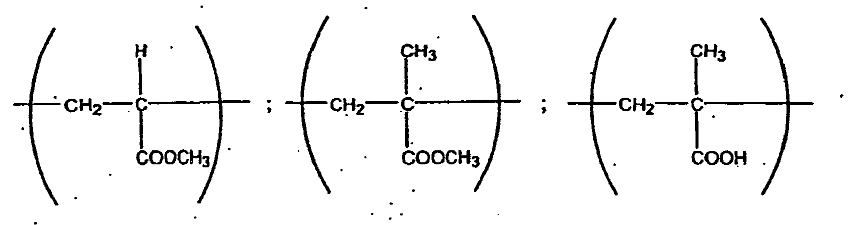

2. Compositions pharmaceutiques selon la revendication 1 pour le traitement d'affections intestinales inflammatoires ou d'irritation.

3. Compositions pharmaceutiques selon la revendication 1, dans lesquelles ledit noyau a) comprend de la maltodextrine en une quantité de 16% en poids du poids total du principe actif.

4. Compositions pharmaceutiques selon la revendication 1, dans lesquelles ledit noyau a) comprend des excipients choisis dans le groupe consistant en liants, agents mouillants, agents anti-agglomération, lubrifiants et leurs mélanges.

5. Compositions pharmaceutiques selon la revendication 4, dans lesquelles le liant est une polyvinylpyrrolidone, l'agent mouillant est du laurylsulfate de sodium, l'agent anti-agglomération est de l'amidon glycolate de sodium, les lubrifiants sont du stéarate de magnésium, du stéarate de magnésium végétal, du talc et leurs mélanges.

6. Compositions pharmaceutiques selon la revendication 1, dans lesquelles la quantité totale d'excipients dans le noyau a) est comprise entre 30% et 40% en poids du poids du principe actif.

7. Compositions pharmaceutiques selon la revendication 1, dans lesquelles la quantité dudit enrobage filmogène b) est comprise entre 5,0% et 7,5% en poids du poids total du noyau.

8. Compositions pharmaceutiques selon la revendication 1, dans lesquelles ledit enrobage filmogène b) a une épaisseur inférieure à 60 µm.

9. Compositions pharmaceutiques selon la revendication 8, dans lesquelles ledit enrobage filmogène b) a une épaisseur comprise dans la gamme de 25 à 50 µm.

10. Compositions pharmaceutiques selon la revendication 8, dans lesquelles ledit enrobage filmogène b) a une épaisseur de 30 µm.

11. Compositions pharmaceutiques selon la revendication 1, dans lesquelles, dans le terpolymère de l'enrobage filmogène b), la quantité d'acrylate de méthyle est comprise entre 60% et 70% en poids, la quantité de méthacrylate de méthyle est comprise entre 20% et 30% en poids et la quantité d'acide méthacrylique est comprise entre 5% et 15% en poids, par rapport au poids total de terpolymère.

12. Compositions pharmaceutiques selon la revendication 11, dans lesquelles le rapport en poids acrylate de méthyle/méthacrylate de méthyle/acide méthacrylique dans ledit terpolymère est de 65 :25 :10.

13. Compositions pharmaceutiques selon la revendication 1, dans lesquelles ledit enrobage filmogène b) contient des excipients choisis dans le groupe consistant en agents plastifiants, agents de polissage, colorants et opacifiants et leurs mélanges.

14. Compositions pharmaceutiques selon la revendication 13, dans lesquelles ledit enrobage filmogène b) contient du citrate de triéthyle en tant qu'agent plastifiant, du polyéthylène glycol 6000 en tant qu'agent de polissage, du dioxyde de titane en tant qu'agent opacifiant, du talc en tant que lubrifiant, tandis que les agents colorants sont choisis dans le groupe consistant en oxyde de fer rouge, oxyde de fer jaune et leurs mélanges.

15. Compositions pharmaceutiques selon la revendication 14, dans lesquelles ledit enrobage filmogène b) contient en tant qu'agent plastifiant du citrate de triéthyle selon un rapport en poids de 1 :20 par rapport au terpolymère d'acrylate de méthyle/méthacrylate de méthyle/acide méthacrylique.

16. Procédé pour la préparation de compositions pharmaceutiques pour administration orale sous forme de comprimé, contenant de la mésalazine ou des sels de celle-ci acceptables du point de vue pharmaceutique en tant que principe actif, comprenant :
a) un noyau comprenant le principe actif et de la maltodextrine en une quantité comprise entre 10% et 20% en poids du poids total du principe actif, et
b) un enrobage filmogène contenant en tant qu'unique polymère filmogène un terpolymère d'acrylate de méthyle/méthacrylate de méthyle/acide méthacrylique,
ledit procédé comprenant les étapes suivantes :
i) mélange du principe actif avec de la maltodextrine, granulation à l'état humide du mélange résultant avec une solution contenant des liants et des agents mouillants, et séchage de la masse granulaire obtenue ;
ii) compression de la masse granulaire telle qu'elle est obtenue dans l'étape i), additionnée éventuellement d'excipients choisis dans le groupe consistant en liants, agents mouillants, agents anti-agglomération, lubrifiants et leurs mélanges ;
iii) addition d'une dispersion aqueuse à 30% de terpolymère d'acrylate de méthyle/méthacrylate de méthyle/acide méthacrylique avec l'agent plastifiant et l'agent de polissage, addition éventuellement à ladite dispersion d'une suspension aqueuse d'agents colorants contenant éventuellement un lubrifiant et un agent opacifiant, pour donner le mélange filmogène mentionné plus haut ;
iv) enduction par pulvérisation des comprimés tels qu'ils sont obtenus dans l'étape ii) ci-dessus avec le mélange tel qu'il est préparé de la façon qui est décrite dans l'étape iii).

17. Procédé selon la revendication 16, dans lequel la dispersion aqueuse à 30% de terpolymère tel qu'il est obtenu dans l'étape iii) est préalablement amenée à neutralité par addition d'une solution aqueuse de NaOH.

## Patentansprüche

1. Pharmazeutische Zusammensetzungen zur oralen Verabreichung in Tablettenform, die Mesalazin oder pharmazeutisch annehmbare Salze hiervon als aktiven Inhaltsstoff enthalten, umfassend:
a) einen Kern, umfassend den aktiven Inhaltsstoff und Maltodextrin in einer Menge zwischen 10 Gew.-% und 20 Gew.-% des Gesamtgewichtes des aktiven Inhaltsstoffes, und
b) einen filmbildenden Überzug, der als einziges filmbildendes Polymeres ein Terpolymeres enthält, das die folgenden monomeren Methylacrylat-, Methylmethacrylat-, Methacrylsäureeinheiten in der Kette hat:

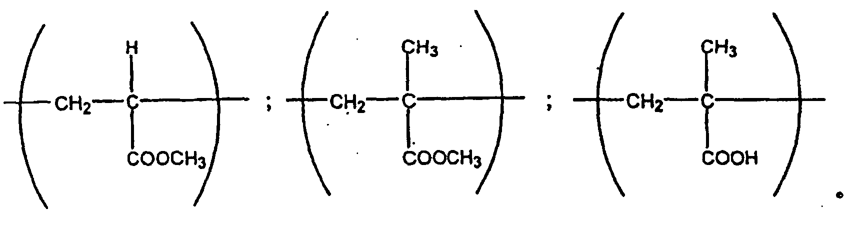

2. Pharmazeutische Zusammensetzungen wie in Anspruch 1 beansprucht für die Behandlung von intestinalen Entzündungs- oder Reizkrankheiten.

3. Pharmazeutische Zusammensetzungen wie in Anspruch 1 beansprucht, in welchen dieser Kern a) Maltodextrin in einer Menge von 16 Gew.-% des Gesamtgewichtes des aktiven Inhaltsstoffes umfaßt.

4. Pharmazeutische Zusammensetzungen wie in Anspruch 1 beansprucht, in welchen dieser Kern a) Trägermittel umfaßt, die aus der Gruppe ausgewählt sind, welche aus Bindemitteln, Benetzungsmitteln, Mitteln gegen Zusammenkleben, Gleitmitteln und Mischungen hiervon besteht.

5. Pharmazeutische Zusammensetzungen wie in Anspruch 4 beansprucht, in welchen das Bindemittel Polyvinylpyrrolidon, ist, das Benetzungsmittel Natriumlaurylsulfat ist, das Mittel gegen Zusammenkleben Natriumstärkeglycolat ist, die Gleitmittel Magnesiumstearat, pflanzliches Magnesiumstearat, Talkum und Mischungen hiervon sind.

6. Pharmazeutische Zusammensetzungen wie in Anspruch 1 beansprucht, in welchen die Gesamtmenge dieser Trägermittel im Kern a) zwischen 30 Gew.-% und 40 Gew.-% des Gewichtes des aktiven Inhaltsstoffes ist.

7. Pharmazeutische Zusammensetzungen wie in Anspruch 1 beansprucht, in welchen die Menge dieses filmbildenden Überzuges b) zwischen 5,0 Gew.-% und 7,5 Gew.-% des Gewichtes des Kerns ist.

8. Pharmazeutische Zusammensetzungen wie in Anspruch 1 beansprucht, in welchen dieser filmbildende Überzug b) eine Dicke niedriger als 60 µm hat.

9. Pharmazeutische Zusammensetzungen wie in Anspruch 8 beansprucht, in welchen dieser filmbildende überzug b) eine Dicke im Bereich von 25 bis 50 µm hat.

10. Pharmazeutische Zusammensetzungen wie in Anspruch 8 beansprucht, in welchen dieser filmbildende Überzug b) eine Dicke von 30 µm hat.

11. Pharmazeutische Zusammensetzungen wie in Anspruch 1 beansprucht, in welchen in dem Terpolymeren des filmbildenden Überzuges b) die Menge von Methylacrylat zwischen 60 Gew.-% und 70 Gew.-% ist, die Menge von Methylmethacrylat zwischen 20 Gew.-% und 30 Gew.-% ist, und die Menge von Methacrylsäure zwischen 5 Gew.-% und 15 Gew.-% ist, bezogen auf das Terpolymergesamtgewicht.

12. Pharmazeutische Zusammensetzungen wie in Anspruch 11 beansprucht, in welchen das Gewichtsverhältnis von Methylacrylat/Methylmethacrylat/Methacrylsäure in diesem Terpolymeren 65:25:10 ist.

13. Pharmazeutische Zusammensetzungen wie in Anspruch 1 beansprucht, in welchen dieser filmbildende Überzug b) Trägermittel enthält, die aus der Gruppe ausgewählt sind, welche aus weichmachenden, polierenden, farbgebenden und opazifierenden Mitteln und Mischungen hiervon besteht.

14. Pharmazeutische Zusammensetzungen wie in Anspruch 13 beansprucht, in welchen dieser filmbildende Überzug b) Triethylcitrat als weichmachendes Mittel, Polyethylenglycol 6000 als polierendes Mittel, Titandioxid als opazifierendes Mittel, Talkum als Gleitmittel enthält, während die farbgebenden Mittel aus der Gruppe ausgewählt sind, welche aus Eisenoxidrot, Eisenoxidgelb und Mischungen hiervon besteht.

15. Pharmazeutische Zusammensetzungen wie in Anspruch 14 beansprucht, in welchen dieser filmbildende Überzug b) als weichmachendes Mittel Triethylcitrat in einem Gewichtsverhältnis von 1:20 zu dem Methacrylsäure/Methylacrylat/Methylmethacrylatterpolmeren enthält.

16. Verfahren für die Herstellung von pharmazeutischen Zusammensetzungen zur oralen Verabreichung in Tablettenform, die Mesalazin oder pharmazeutische annehmbare Salze hiervon als aktiven Inhaltsstoff enthalten, umfassend:
a) einen Kern, umfassend den aktiven Inhaltsstoff und Maltodextrin in einer Menge zwischen 10 Gew.-% und 20 Gew.-% des Gesamtgewichtes des aktiven Inhaltsstoffes, und
b) einen filmbildenden Überzug, der als einziges filmbildendes Polymeres ein Methylacrylat/Methylmethacrylat/Methacrylsäureterpolymeres enthält,
wobei dieses Verfahren die folgenden Stufen umfaßt:
i) Mischen des aktiven Inhaltsstoffes mit Maltodextrin, Naßgranulieren des resultierenden Gemisches mit einer Lösung, welche Bindemittel und Benetzungsmittel enthält, und Trocknen der erhaltenen Granulatmasse,
ii) Pressen der Granulatmasse, wie in Stufe i) erhalten, wahlweise versetzt mit Trägermitteln, die aus der Gruppe ausgewählt sind, welche aus Bindemitteln, Benetzungsmitteln, Mitteln gegen Zusammenkleben, Gleitmitteln und Mischungen hiervon besteht,
iii) Versetzen einer wässrigen 30%igen Dispersion von Methacrylsäure/Methylacrylat/Methylmethacrylatterpolymerem mit den weichmachenden und polierenden Mitteln, wahlweise Versetzen dieser Dispersion mit einer wässrigen Dispersion von farbgebenden Mitteln, die wahlweise ein Gleitmittel und ein opazifierendes Mittel enthalten, zum Erhalt der zuvor genannten filmbildenden Mischung,
iv) Sprühbeschichten der in Stufe ii) oben erhaltenen Tabletten mit der Mischung, die wie in Stufe iii) beschrieben hergestellt wurde.

17. Verfahren wie in Anspruch 16 beansprucht, bei welchem die wässrige 30%ige Dispersion von Terpolymerem für Stufe iii) zuvor auf Neutralität durch Zugabe einer wässrigen NaOH-Lösung gebracht wurde.
